# EUROPEAN PATENT SPECIFICATION

(11) **EP 3 381 894 B1**
(45) Date of publication and mention of the grant of the patent: **18.08.2021**
(21) Application number: 18165201.7
(22) Date of filing: 29.03.2018
(51) Int. Cl.: C07C 319/26, C07C 321/14, C08G 18/38, B29D 11/00, G02B 1/04, C07C 319/14, C07C 323/64, C08G 18/24, C08G 18/76

(54) **POLYTHIOL COMPOSITION WITH IMPROVED LONG-TERM STORAGE STABILITY AND OPTICAL LENSES USING SAME**
POLYTHIOLZUSAMMENSETZUNG MIT VERBESSERTER LANGZEITLAGERSTABILITÄT UND OPTISCHE LINSEN DAMIT
COMPOSITION DE POLYTHIOL AYANT UNE MEILLEURE STABILITÉ AU STOCKAGE À LONG TERME ET LENTILLES OPTIQUES L'UTILISANT

(30) Priority: 31.03.2017 KR 20170041824; 24.08.2017 KR 20170107197
(43) Date of publication of application: 03.10.2018
(73) Proprietor: SKC Co., Ltd., Suwon-si, Gyeonggi-do 16336 (KR)
(72) Inventor: SHIN, Junghwan, 16663 Gyeonggi-do (KR); HONG, Seung Mo, 21994 Incheon (KR); SHIM, Jongmin, 18319 Gyeonggi-do (KR); HAN, Hyuk Hee, 16500 Gyeonggi-do (KR); MYUNG, Jung Hwan, Gyeonggi-do 14963 (KR); SEO, Hyeon Myeong, 44679 Ulsan (KR); SONG, Yu Jeong, 03370 Seoul (KR)
(74) Representative: Dehns

(56) References cited:
- EP-A1- 2 011 785
- EP-A1- 2 910 592
- EP-A2- 0 810 251
- WO-A1-02/051911

## Description

### Technical Field

The invention relates to a polythiol composition used for the preparation of an optical material. More specifically, the invention relates to a polythiol composition with improved long-term storage stability, a polymerizable composition using the same, and an optical material, particularly an optical lens.

### Background Art

Plastic optical materials are lightweight, hardly breakable, and excellent in dyeability as compared with optical materials made of inorganic materials such as glass. Therefore, plastic materials are widely used as optical materials for eyeglass lenses, camera lenses, and the like. In recent years, there has been an increased demand for higher performance of optical materials, particularly in terms of high refractive index, high Abbe number, low specific gravity, high heat resistance, high impact resistance, and the like.

Polythiourethane among such plastic optical materials is widely used as an optical material by virtue of its excellent optical characteristics and mechanical properties. Polythiourethane may be prepared by reacting a polythiol and an isocyanate. Lenses made from polythiourethane are widely used by virtue of their high refractive index, lightweight, and relatively high impact resistance.

A polythiol used for the preparation of optical lenses with a high refractive index has a thiol group at the terminal ends thereof. Although the thiol group is advantageous for improving such optical properties as refractive index, it may bond each other due to its high reactivity or easily react with an external chemical, thereby generating a byproduct.

If optical lenses are prepared from a polythiol whose quality is deteriorated due to such a byproduct, the optical properties of the lenses may be adversely affected. Therefore, considerable care must be taken in the preparation and storage of a polythiol in order to prevent this problem.

EP 2 910 592 discloses a polymerizable composition which comprises a polythiol compound and an enic compound having a phenylthio backbone as monomer components, an acid that serves as a stabilizing agent, and a free-radical polymerization inhibitor. WO 02/051911 discloses a polymerizable composition comprising at least one polymerizable monomer which has at least one episulfide functionality and at least one (alkoxyphenyl)phosphine polymerization catalyst. EP 2 011 785 discloses a process for producing a polythiol compound for an optical material, the method including reacting 2-mercaptoethanol with an epihalohydrin compound to give polyalcohol, and preparing a polythiol compound through the polyalcohol, wherein a content of bis(2-hydroxyethyl) disulfide in the 2-mercaptoethanol is 0.5 wt% or less.

### Disclosure of Invention

### Technical Problem

The present inventors have carefully studied various process factors that may cause a deterioration in the quality of a polythiol composition during long-term storage after the preparation of the polythiol composition, particularly paying attention to the fact that oxygen present in the final polythiol composition is stimulated by external light or temperature, thereby generating active oxygen. Especially, the present inventors have discovered that active oxygen directly reacts with a thiol group or has a significant impact on the reactivity of a thiol group with other functional groups; therefore, it causes a decrease in the number of functional groups, a change in color, a change in viscosity, and the like, so that it has a great impact on the quality of a lens finally produced.

Therefore, it is intended to prevent the generation of a byproduct or the deterioration in the physical properties of a polythiol composition that may be caused by active oxygen during storage thereof by way of controlling the content of active oxygen in the polythiol composition to a predetermined level or lower through the following embodiments, to thereby enhance the long-term storage stability of the polythiol composition.

In the meantime, oxygen may be introduced not only through the raw materials used in the process of synthesizing a polythiol but also through contact of the raw materials with a solvent, washing water, or air. However, an attempt to remove oxygen from all of the raw materials for a polythiol, washing water, and the like, or to block oxygen in all of the process steps would significantly increase the production cost of a polythiol. Further, it is difficult to make such attempt successful in reality.

Accordingly, it is intended through the following invention to provide a novel process, which is capable of greatly improving the long-term storage stability of a polythiol composition and the quality of an optical lens finally produced therefrom by way of efficiently and economically controlling the content of active oxygen in the final polythiol composition through a single step of removing active oxygen subsequent to the synthesis of the polythiol.

### Solution to Problem

According to the invention, there is provided a polythiol composition, which comprises a bi- or higher-functional polythiol; and a reducing agent. The polythiol composition comprises the reducing agent in an amount of 500 ppm or less, and the reducing agent is at least one selected from the group consisting of lithium aluminum hydride (LiAlH₄), sodium amalgam (Na(Hg)), zinc amalgam (Zn(Hg)), diborane (B₂H₆), sodium borohydride (NaBH4), iron (II) sulfate (FeSO₄), tin (II) chloride (SnCl₂), sodium dithionate (Na₂S₂O₆), sodium thiosulfate (Na₂S₂O₃), ammonium thiosulfate ((NH₄)₂S₂O₃), diisobutyl aluminum hydride (DIBAL-H), formic acid (HCOOH), ascorbic acid (C₆H₈O₆), reducing sugars, phosphites, hypophosphites, dithiothreitol (DTT), tris-2-carboxyethylphosphine hydrochloride (TCEP), 2,2-diphenyl-1-picrylhydrazide (DPPH), butylated hydroxytoluene (BHT), di-tert-butyl-4-methylphenol, and 2,4-dimethyl-6-tert-butylphenol.

There is also provided a process for preparing the above-mentioned polythiol composition, which comprises preparing a composition comprising a bi- or higher-functional polythiol; and treating the composition with a reducing agent. The treatment with the reducing agent is carried out by introducing the reducing agent into the composition and then removing the reducing agent from the composition, and the reducing agent is at least one selected from the group consisting of lithium aluminum hydride (LiAlH₄), sodium amalgam (Na(Hg)), zinc amalgam (Zn(Hg)), diborane (B₂H₆), sodium borohydride (NaBH₄), iron (II) sulfate (FeSO₄), tin (II) chloride (SnCl₂), sodium dithionate (Na₂S₂O₆), sodium thiosulfate (Na₂S₂O₃), ammonium thiosulfate ((NH₄)₂S₂O₃), diisobutyl aluminum hydride (DIBAL-H), formic acid (HCOOH), ascorbic acid (C₆H₈O₆), reducing sugars, phosphites, hypophosphites, dithiothreitol (DTT), tris-2-carboxyethylphosphine hydrochloride (TCEP), 2,2-diphenyl-1-picrylhydrazide (DPPH), butylated hydroxytoluene (BHT), di-tert-butyl-4-methylphenol, and 2,4-dimethyl-6-tert-butylphenol.

There is also provided a polymerizable composition, which comprises the polythiol composition; and an isocyanate.

There is also provided an optical material, which comprises a polythiourethane formed by polymerizing the polythiol composition and an isocyanate.

There is also provided a process for preparing an optical lens, which comprises preparing a polythiol composition as described above, and mixing the composition with an isocyanate and thermally curing the mixture in a mold.

### Advantageous Effects of Invention

According to an aspect of the disclosure, it is possible to prevent the generation of a byproduct or the deterioration in the physical properties of a polythiol composition that may be caused by active oxygen during storage thereof by way of controlling the content of active oxygen in the polythiol composition to 200 ppm or less. As a result, it is possible to enhance the long-term storage stability of a polythiol composition after it has been synthesized until it is used in the polymerization reaction.

In particular, according to another aspect of the disclosure, it is possible to reduce the content of active oxygen in a polythiol composition finally prepared in a more efficient, convenient, and economical way by removing active oxygen from the polythiol composition in s step subsequent to the synthesis of the polythiol, as compared with the method of removing active oxygen from all of the raw materials to be used in the synthesis of the polythiol, washing water, and the like.

According to the invention, it is possible to efficiently reduce the content of active oxygen in a polythiol composition to a very low level of 10 ppm or less through a simple method of treating the polythiol composition immediately after the synthesis thereof with a reducing agent. In this case, any changes in color, viscosity, and byproducts of the polythiol composition have been hardly observed even after storage for 6 months.

Therefore, even if a polythiol composition prepared according to the invention is used after storage thereof for a long period of time for sale after the preparation thereof, the polythiol composition can be polymerized with an isocyanate to produce a polythiourethane having excellent properties in terms of refractive index, Abbe number, transparency, glass transition temperature, yellow index, and the like. It can be advantageously used for an optical material such as eyeglass lenses, camera lenses, and the like.

### Detailed Description for Carrying out the Invention

In the following embodiments or aspects, the term "polythiol" refers to a compound having two or more thiol groups (-SH) in the molecule.

Further, in the following embodiments or aspects, the term "polythiol composition" refers to a composition comprising a polythiol as a main component and other components in small amounts.

In addition, in the following embodiments or aspects, the term "active oxygen" refers to all reactive byproducts of oxygen inclusive of highly reactive oxygen, as is well known in the art. In particular, active oxygen includes oxygen species that cause a side reaction in a polythiol composition, thereby deteriorating the physical properties of the polythiol composition and degrading the optical properties of an optical material produced therefrom. Representative examples of active oxygen include, but are not limited to, hydrogen peroxide (H₂O₂), superoxide ion (O₂⁻), a hydroxyl radical (·OH), singlet oxygen, and the like.

According to an aspect of the disclosure, there is provided a process for preparing a polythiol composition, which comprises adjusting the content of active oxygen in a composition comprising a bi- or higher-functional polythiol to 200 ppm or less.

According to the invention, there is provided a process for preparing a polythiol composition, which comprises preparing a composition comprising a bi- or higher-functional polythiol; and treating the composition with a reducing agent. The treatment with the reducing agent is carried out by introducing the reducing agent into the composition and then removing the reducing agent from the composition, and the reducing agent is at least one selected from the group consisting of lithium aluminum hydride (LiAlH₄), sodium amalgam (Na(Hg)), zinc amalgam (Zn(Hg)), diborane (B₂H₆), sodium borohydride (NaBH₄), iron (II) sulfate (FeSO₄), tin (II) chloride (SnCl₂), sodium dithionate (Na₂S₂O₆), sodium thiosulfate (Na₂S₂O₃), ammonium thiosulfate ((NH₄)₂S₂O₃), diisobutyl aluminum hydride (DIBAL-H), formic acid (HCOOH), ascorbic acid (C₆H₈O₆), reducing sugars, phosphites, hypophosphites, dithiothreitol (DTT), tris-2-carboxyethylphosphine hydrochloride (TCEP), 2,2-diphenyl-1-picrylhydrazide (DPPH), butylated hydroxytoluene (BHT), di-tert-butyl-4-methylphenol, and 2,4-dimethyl-6-tert-butylphenol.

According to an aspect of the disclosure, the process comprises (1) preparing a composition comprising a bi- or higher-functional polythiol; and (2) reducing the content of active oxygen in the composition to 200 ppm or less.

According to an embodiment, the process comprises (1) preparing a composition comprising a bi- or higher-functional polythiol; and (2) treating the composition with a reducing agent to reduce the content of active oxygen in the composition to 200 ppm or less.

In the above step (1), a composition comprising a bi- or higher-functional polythiol is prepared.

For this purpose, a polythiol may be synthesized from raw materials, during which a trace amount of byproducts may be produced, thereby obtaining a polythiol composition comprising them.

Here, the polythiol may be synthesized in a conventional manner known in the art, and it is not particularly limited in terms of raw materials, reactants, and synthesis conditions.

As an example, the synthesis of the polythiol may be carried out by reacting a polyol with a sulfur compound.

In such event, the polyol may have at least two terminal OH groups and may be commercially purchased or directly synthesized. For example, the polyol may be prepared in the form of a tetraol by reacting a chlorodiol with a metal sulfide. Here, the chlorodiol may be prepared by reacting epichlorohydrin with 2-mercaptoethanol in the presence of triethylamine. Further, the metal sulfide may be Na₂Sₙ.xH₂O (n is an integer of 1 to 8, and x is 5 or 9) or S(MgBr)₂.

As a specific example, the synthesis of a polythiol may be carried out by reacting a polyol with thiourea, followed by hydrolysis of the resultant to produce a polythiol.

That is, the polyol is reacted with thiourea to prepare an isothiouronium salt, and the isothiouronium salt is hydrolyzed to synthesize a polythiol.

In the above method, the polyol may be first mixed with thiourea and refluxed under an acidic condition to obtain an isothiouronium salt. In such event, 1 to 3 equivalents, more specifically 1 to 2 equivalents, of thiourea may be reacted per 1 equivalent of the OH group in the polyol. Further, the reflux temperature may be 60 to 130°C, more preferably 90 to 120°C. Also, the reflux time may be 2 to 24 hours, more specifically 6 to 12 hours.

Thereafter, the isothiouronium salt may be hydrolyzed under a basic condition to synthesize a polythiol. Such a basic compound as sodium hydroxide, potassium hydroxide, sodium carbonate, ammonia, and the like may be used for the basic condition. The basic compound may be employed in an amount of 1.0 to 2.5 equivalents, more specifically 1.3 to 1.8 equivalents, per 1 equivalent of the isothiouronium salt. For example, the basic compound may be employed in the form of an aqueous solution. An organic solvent may be added before the basic compound is employed. The amount of the organic solvent added may be 0.1 to 3.0 times, more specifically 0.2 to 2.0 times, the amount of the isothiouronium salt solution. Examples of the organic solvent include toluene, xylene, chlorobenzene, dichlorobenzene, and the like. Toluene is preferred for the purpose of suppressing the formation of byproducts. The reaction temperature for the hydrolysis may be 10 to 130°C, more specifically 30 to 80°C. The time for the hydrolysis may be 0.1 to 8 hours, more specifically 2 to 6 hours.

According to a preferred example, the above step (1) may comprise (1a) reacting a chlorodiol compound with a metal-based sulfide to prepare a tetraol compound; (1b) reacting the tetraol compound with thiourea and hydrochloric acid to prepare an isothiouronium salt; and (1c) hydrolyzing the isothiouronium salt with a basic aqueous solution to synthesize a polythiol.

The reaction mixture prepared as a result may be subjected to purification. Specifically, acid washing and several times of water washing may be performed, or alkaline washing may be performed after acid washing and water washing. Impurities and the like can be removed through the washing step, and the color of the polythiol can be improved, resulting in an improvement in the color of the optical material obtained therefrom as well. The acid washing may be carried out by adding hydrochloric acid to the reaction mixture. The concentration of hydrochloric acid is suitably 30 to 36%, and the temperature for the acid washing may be 20 to 60°C, more specifically 20 to 40°C. In addition, distilled water having an oxygen concentration of 5 mg/L or less may be used in the water washing. The alkaline washing may be performed by adding an alkaline aqueous solution, followed by stirring the reaction mixture at 20 to 40°C for 10 minutes to 2 hours.

The polythiol synthesized through the procedures as described above may be a bi- or higher-functional polythiol. For example, it may be a polythiol having 2 to 10, 2 to 8, 2 to 6, or 2 to 4 thiol groups in the molecule.

Further, the polythiol may be a single polythiol or a mixture two or more polythiols.

Specific examples of the polythiol include 3,3'-thiobis[2-[(2-mercaptoethyl)thio]-1-propanethiol, bis(2-(2-mercaptoethylthio)-3-mercaptopropyl) sulfide, 4-mercaptomethyl-1,8-dimercapto-3,6-dithiaoctane, 2,3-bis(2-mercaptoethylthio)propane-1-thiol, 2,2-bis(mercaptomethyl)-1,3-propanedithiol, bis(2-mercaptoethyl) sulfide, tetrakis(mercaptomethyl)methane, 2-(2-mercaptoethylthio)propane-1,3-dithiol, 2-(2,3-bis(2-mercaptoethylthio)propylthio)ethanethiol, bis(2,3-dimercaptopropanyl) sulfide, bis(2,3-dimercaptopropanyl) disulfide, 1,2-bis[(2-mercaptoethyl)thio)-3-mercaptopropane, 1,2-bis(2-(2-mercaptoethylthio)-3 -mercaptopropylthio)ethane, 2-(2-mercaptoethylthio)-3-2-mercapto-3-[3-mercapto-2-(2-mercaptoethylthio)-propylthio]propylthio-propane-1-thiol, 2,2-bis-(3-mercapto-propionyloxymethyl)-butyl ester, 2-(2-mercaptoethylthio)-3-(2-(2-[3-mercapto-2-(2-mercaptoethylthio)-propylthio] ethylthio)ethylthio)propane-1-thiol, (4R,11S)-4,11-bis(mercaptomethyl)-3,6,9,12-tetrathiatetradecane-1,14-dithiol, (S)-3-((R-2,3-dimercaptopropyl)thio)propane-1,2-dithiol, (4R,14R)-4,14-bis(mercaptomethyl)-3,6,9,12,15-pentathiaheptane-1,17-dithiol, (S)-3-((R-3-mercapto-2-((2-mercaptoethyl)thio)propylthio)propylthio)-2-((2-mercaptoethyl)thio)propane-1-thiol, 3,3'-dithiobis(propane-1,2-dithiol), (7R,11S)-7,11-bis(mercaptomethyl)-3,6,9,12,15-pentathiaheptadecane-1,17-dithiol, (7R,12S)-7,12-bis(mercaptomethyl)-3,6,9,10,13,16-hexathiaoctadecane-1,18-dithiol, 5,7-dimercaptomethyl-1,11-dimercapto-3,6,9-trithiaundecane, 4,7-dimercaptomethyl-1,11-dimercapto-3,6,9-trithiaundecane, 4,8-dimercaptomethyl-1,11-dimercapto-3,6,9-trithiaundecane, pentaerythritol tetrakis(3-mercaptopropionate), trimethylolpropane tris(3-mercaptopropionate), pentaerythritol tetrakis(2-mercaptoacetate), bispentaerythritol ether hexakis(3-mercaptopropionate), 1,1,3,3-tetrakis(mercaptomethylthio)propane, 1,1,2,2-tetrakis(mercaptomethylthio)ethane, 4,6-bis(mercaptomethylthio)-1,3-dithiane, pentaerythritol tetrakis(2-mercaptoacetate), pentaerythritol tetrakis(3-mercaptopropionate), and 2-(2,2-bis(mercaptodimethylthio)ethyl)- 1,3-dithiane.

Preferably, the polythiol may be a tri-, tetra- or higher-functional polythiol.

In addition, a trace amount of components inevitably generated in the synthesis of a polythiol is mixed with the polythiol to constitute the polythiol composition.

Further, the polythiol composition obtained in the above step (1) may comprise a solvent, which may be a portion of the solvent used in the synthesis of the polythiol as described above that remains in the composition without being removed therefrom. The polythiol composition may comprise 10 to 200 parts by weight or 30 to 100 parts by weight of the solvent based on 100 parts by weight of the polythiol.

The polythiol composition may be used in the next step (2) as it is without removing the organic solvent.

In the above step (2), the content of active oxygen in the composition obtained in the previous step (1) may be reduced to 200 ppm or less.

In general, any organic material, inclusive of a polythiol composition, contains a certain amount of oxygen as dissolved oxygen peculiar to the material under the specific conditions of temperature and pressure. Such dissolved oxygen may continuously cause side reactions unless it is deliberately removed during the preparation of the polythiol composition.

In particular, the oxygen dissolved in the polythiol composition is activated by an external stimulus such as light or temperature, and the active oxygen thus generated directly reacts with the thiol group or significantly affects the reactivity between the thiol group and other functional groups. Specifically, if active oxygen is contained in a polythiol composition at a certain level or higher, a side reaction such as the formation of a disulfide may take place, resulting in a decrease in the number of functional groups, a change in color, a change in viscosity, and the like. It may also have an impact on the activity of the thiol group, thereby affecting the workability and the curing rate in the reaction with an isocyanate or the like. As a result, it may cause a serious defect in the quality of a lens finally produced.

More specifically, if active oxygen is present in a polythiol composition, it may generate various byproducts such as a disulfide as illustrated in the following Reaction Schemes, cause a decrease in the number of the thio group, an increase in viscosity, as well as a change in color, and affect the reactivity of the composition.

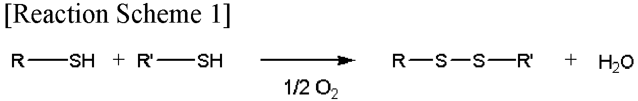

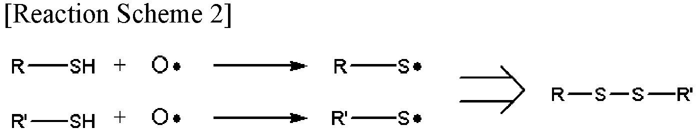

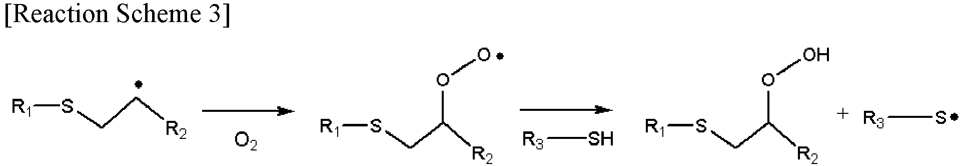

Therefore, in order to maintain the quality of a polythiol composition, it is necessary to always control the content of active oxygen in the polythiol composition to a certain level or lower.

However, oxygen may be introduced not only through the raw materials used in the process of synthesizing a polythiol but also through contact of the raw materials with a solvent, washing water, or air. Thus, in order to completely remove oxygen, it is necessary to remove oxygen from all of the raw materials for a polythiol, washing water, and the like, and to block oxygen in all of the process steps such as synthesis, packaging, and the like. An effort to remove or manage active oxygen from all of the raw materials and in all of the process steps would greatly increase the production cost. And it is difficult to make such attempt successful in reality in many cases. Further, there still has been a problem that the efficiency of removing active oxygen from a polythiol composition is not so high in the methods attempted so far, so that a certain amount of active oxygen is always present in the polythiol composition even after the treatment.

Accordingly, in the process according to the invention, the content of active oxygen in a final polythiol composition is efficiently and economically controlled through a single step of removing active oxygen subsequent to the synthesis of the polythiol.

In an aspect of the disclosure, the content of active oxygen may be reduced by introducing an inert gas into the polythiol composition, to thereby physically remove active oxygen from the polythiol composition. In such event, the inert gas may be introduced under the conditions of a reduced pressure and a temperature of room temperature to 80°C.

According to the invention, the content of active oxygen is reduced by treating the polythiol composition with a reducing agent. Such treatment with a reducing agent can reduce the content of active oxygen in the composition to 200 ppm or less.

The treatment with a reducing agent is carried out by introducing the reducing agent into the polythiol composition and then removing the reducing agent from the polythiol composition.

Here, the reducing agent may be added in an amount of 0.001 to 10 parts by weight, 0.01 to 5 parts by weight, or 0.1 to 5 parts by weight based on 100 parts by weight of the polythiol composition.

Further, the reduction reaction with a reducing agent may be carried out at a temperature of room temperature to 80°C or a temperature of 40°C to 60°C. Further, the reduction reaction may be carried out for 30 minutes to 5 hours or 30 minutes to 3 hours.

As a preferable example, the treatment with a reducing agent may be carried out by adding the reducing agent in an amount of 0.01 to 5 parts by weight based on 100 parts by weight of the polythiol composition, performing a reduction reaction at a temperature of room temperature to 80°C for 30 minutes to 3 hours, and removing the reducing agent from the polythiol composition.

The reducing agent is capable of removing active oxygen from a polythiol composition using an oxidation or reduction reaction. The reducing agent is at least one selected from the group consisting of lithium aluminum hydride (LiAlH₄), sodium amalgam (Na(Hg)), zinc amalgam (Zn(Hg)), diborane (B₂H₆), sodium borohydride (NaBH4), iron (II) sulfate (FeSO₄), tin (II) chloride (SnCl₂), sodium dithionate (Na₂S₂O₆), sodium thiosulfate (Na₂S₂O₃), ammonium thiosulfate ((NH₄)₂S₂O₃), diisobutyl aluminum hydride (DIBAL-H), ascorbic acid (C₆H₈O₆), reducing sugars, phosphites, hypophosphites, dithiothreitol (DTT), tris-2-carboxyethylphosphine hydrochloride (TCEP), 2,2-diphenyl-1-picrylhydrazide (DPPH), butylated hydroxytoluene (BHT), di-tert-butyl-4-methylphenol, and 2,4-dimethyl-6-tertbutylphenol.

Sodium borohydride (NaBH4) and sodium thiosulfate (Na₂S₂O₃) among the above are favorable as the reducing agent from the viewpoint of work convenience such as compatibility with a polythiol and solubility in water.

After the treatment of the polythiol composition with a reducing agent as described above, the reducing agent is removed from the polythiol composition.

If the reducing agent is in a solid phase, it may be removed by filtration using a filter. In such event, depressurized or pressurized filtration using a membrane filter or a cartridge filter may be used. The pore size of the filter is preferably 5 µm or less, more specifically 2 µm or less.

In addition, if the reducing agent is in a liquid phase, it may be removed by fractional distillation or the like, in which the degree of depressurization and the temperature may be appropriately selected in view of the boiling point and the decomposition temperature of the polythiol thus prepared.

The removal of the reducing agent is carried out such that the amount of the reducing agent in the polythiol composition is 500 ppm or less.

After the treatment with a reducing agent, a trace amount of the reducing agent is left in the polythiol composition without completely removing it. For example, the removal of the reducing agent may be carried out such that the reducing agent is present in an amount of 1 to 500 ppm or 1 to 100 ppm based on the weight of the polythiol composition.

The residual reducing agent can prevent a deterioration by oxygen that may be introduced during long-term storage or delivery, to thereby improve the storage stability of the composition. Further, if the amount of the residual reducing agent is within the above preferable range, it may be advantageous to prevent phase separation, precipitation, and discoloration of the polythiol composition and a deterioration in transparency and color of a lens, which may be caused by an excessive amount of the reducing agent.

In addition, if the reducing agent is introduced to the polythiol composition as obtained in the previous step (1) from which the organic solvent used in the synthesis of the polythiol has not been removed, the organic solvent may be removed after the treatment with the reducing agent. In such event, the organic solvent may be removed at a reduced pressure and a temperature of 30 to 100°C or 50 to 80°C. The degree of depressurization may be appropriately adjusted according to the temperature.

As a specific example, the polythiol composition contains an organic solvent, the treatment with a reducing agent is carried out by introducing the reducing agent to the polythiol composition, and the organic solvent and the reducing agent may then be removed from the polythiol composition.

The polythiol composition subjected to the treatment with a reducing agent as described above may be measured for the content of active oxygen. If necessary, the treatment with a reducing agent may be repeated.

Here, the content of active oxygen may be measured by a known method, for example, by a method comprising (1) oxidizing active oxygen and Fe²⁺ ions (or ferrous ions) in the polythiol composition; (2) reacting the Fe³⁺ ions thus generated by the oxidation with ammonium thiocyanate to obtain a ferric thiocyanate complex; (3) measuring the absorbance thereof using a spectrophotometer; and (4) converting the content of active oxygen in the polythiol composition using a calibration curve of the change in absorbance with respect to the content of active oxygen.

The final polythiol composition thus prepared (i.e., a polythiol composition having a reduced content of active oxygen) may be stored in a sealed container filled with an inert gas at 20°C or less, for example, at 10 to 20°C.

In addition, according to an aspect of the disclosure, there is provided a polythiol composition, which comprises a bi- or higher-functional polythiol, wherein the content of active oxygen in the composition is 200 ppm or less.

The polythiol may be a bi- or higher-functional polythiol. For example, it may be a polythiol having 2 to 10, 2 to 8, 2 to 6, or 2 to 4 thiol groups in the molecule.

Further, the polythiol composition may comprise a single polythiol or a mixture of two or more polythiols. The specific types of the polythiol available for the composition are as exemplified above.

The polythiol composition according to the aspect has a content of active oxygen of 200 ppm or less. More preferably, the content of active oxygen in the polythiol composition may be 100 ppm or less, 50 ppm or less, 30 ppm or less, 20 ppm or less, or 10 ppm or less.

The lower limit of the content of active oxygen is not particularly limited, but it may be, for example, 0 ppm, more than 0 ppm, 0.1 ppm or more, or 1 ppm or more.

The content of the bi- or higher-functional polythiol in the polythiol composition may be 60% by weight or more, 70% by weight or more, 80% by weight or more, or 90% by weight or more.

For example, the content of the bi- or higher-functional polythiol in the polythiol composition may be 60 to 99.9% by weight, 70 to 99.9% by weight, or 80 to 99.9% by weight.

As a preferred example, the polythiol composition may comprise 70 to 99.9% by weight of a tri- or higher-functional polythiol, wherein the content of active oxygen may be 100 ppm or less.

The polythiol composition may be in a liquid or solid phase at room temperature. In the case of a solid phase, the polythiol composition may be heated to a melting point or higher, if necessary.

The polythiol composition has excellent long-term storage stability by virtue of a reduced content of active oxygen.

For example, the polythiol composition may have a b* value according to the Lab color space changed within 1.0 relative to the initial value after storage for 6 months at 20°C or lower (e.g., 10 to 20°C). More specifically, the polythiol composition may have a b* value according to the Lab color space changed within 0.7, within 0.5, or within 0.3, relative to the initial value after storage for 6 months at 20°C or lower (e.g., 10 to 20°C).

In addition, the polythiol composition may have a viscosity changed within 20% relative to the initial value after storage for 6 months at 20°C or lower (e.g., 10 to 20°C). More specifically, the polythiol composition may have a viscosity changed within 15%, within 10%, or within 5%, relative to the initial value after storage for 6 months at 20°C or lower (e.g., 10 to 20°C).

Further, the polythiol composition may have a content of byproducts changed within 10% relative to the initial value after storage for 6 months at 20°C or lower (e.g., 10 to 20°C). More specifically, the polythiol composition may have a content of byproducts changed within 8% or within 5%, relative to the initial value after storage for 6 months at 20°C or lower (e.g., 10 to 20°C). Here, the content of byproducts may be calculated as a sum of the components that have a shorter elution time (i.e., that have a larger molecular weight) than that of the polythiol as a main component in gel permeation chromatography (GPC).

As a preferred example, the polythiol composition may have a content of byproducts changed within 10% relative to the initial value after storage for 6 months at 20°C or lower. Further, in such event, the polythiol composition may have a b* value according to the Lab color space changed within 1.0 relative to the initial value and a viscosity changed within 20% relative to the initial value after storage for 6 months at 20°C or lower.

According to the invention, there is provided a polythiol composition, which comprises a bi- or higher-functional polythiol; and a reducing agent. In addition, according to a preferred embodiment, there is provided a polythiol composition, which comprises a bi- or higher-functional polythiol; and a reducing agent, wherein the content of active oxygen in the composition is 200 ppm or less. The reducing agent in the polythiol composition may prevent a deterioration by oxygen that may be introduced during long-term storage or transportation of the composition, to thereby improve the storage stability of the composition.

As described above, the polythiol composition comprises a trace amount of a reducing agent. Specifically, it comprises 500 ppm or less, for example, 1 to 500 ppm, or 1 to 100 ppm, of a reducing agent. As the amount of a reducing agent in the polythiol composition is within the above range, it is advantageous to prevent phase separation, precipitation, and discoloration of the polythiol composition and a deterioration in transparency and color of a lens, which may be caused by an excessive amount of the reducing agent.

In addition, there is provided a polymerizable composition, which comprises the polythiol composition according to the invention; and an isocyanate.

The polymerizable composition comprises a polythiol composition, which comprises a bi- or higher-functional polythiol and a reducing agent; and an isocyanate. The reducing agent is present in an amount of 500 ppm or less, and the reducing agent is at least one selected from the group consisting of lithium aluminum hydride (LiAlH₄), sodium amalgam (Na(Hg)), zinc amalgam (Zn(Hg)), diborane (B₂H₆), sodium borohydride (NaBH4), iron (II) sulfate (FeSO₄), tin (II) chloride (SnCl₂), sodium dithionate (Na₂S₂O₆), sodium thiosulfate (Na₂S₂O₃), ammonium thiosulfate ((NH₄)₂S₂O₃), diisobutyl aluminum hydride (DIBAL-H), formic acid (HCOOH), ascorbic acid (C₆H₈O₆), reducing sugars, phosphites, hypophosphites, dithiothreitol (DTT), tris-2-carboxyethylphosphine hydrochloride (TCEP), 2,2-diphenyl-1-picrylhydrazide (DPPH), butylated hydroxytoluene (BHT), di-tert-butyl-4-methylphenol, and 2,4-dimethyl-6-tert-butylphenol.

The polymerizable composition may comprise the polythiol composition and the isocyanate in a mixed state or in a separated state. That is, the polythiol composition and the isocyanate in the polymerizable composition may be in a state of being compounded in contact with each other or separated from each other so as not to contact each other.

The molar ratio of SH group/NCO group in the polymerizable composition may be 0.5 to 3.0, more specifically 0.8 to 1.3.

The isocyanate may be a conventional one commonly used for the synthesis of polythiourethane.

Specifically, the isocyanate may be selected from the group consisting of an aliphatic isocyanate-based compound such as isophorone diisocyanate, dicyclohexylmethane-4,4-diisocyanate, hexamethylene diisocyanate, 2,2-dimethyl pentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecatriisocyanate, 1,3,6-hexamethylene triisocyanate, 1,8-diisocyanato-4-isocyanatomethyloctane, bis(isocyanatoethyl) carbonate, bis(isocyanatoethyl) ether, 1,2-bis(isocyanatomethyl) cyclohexane, 1,3-bis(isocyanatomethyl) cyclohexane, 1,4-bis(isocyanatomethyl) cyclohexane, dicyclohexylmethane diisocyanate, cyclohexane diisocyanate, methylcyclohexane diisocyanate, dicyclohexyldimethylmethane isocyanate, 2,2-dimethyldicyclohexylmethane isocyanate, bis(isocyanatoethyl) sulfide, bis(isocyanatopropyl) sulfide, bis(isocyanatohexyl) sulfide, bis(isocyanatomethyl) sulfone, bis(isocyanatomethyl) disulfide, bis(isocyanatopropyl) disulfide, bis(isocyanatomethylthio)methane, bis(isocyanatoethylthio)methane, bis(isocyanatoethylthio)ethane, bis(isocyanatomethylthio)ethane, 1,5-diisocyanato-2-isocyanatomethyl-3-thiapentane, 2,5-diisocyanatothiophene, 2,5-bis(isocyanatomethyl)thiophene, 2,5-diisocyanatotetrahydrothiophene, 2,5-bis(isocyanatomethyl)tetrahydrothiophene, 3,4-bis(isocyanatomethyl)tetrahydrothiophene, 2,5-diisocyanato- 1,4-dithiane, 2,5-bis(isocyanatomethyl)- 1,4-dithiane, 4,5-diisocyanato- 1,3-dithiolane, 4,5-bis(isocyanatomethyl)-1,3-dithiolane, 4,5-bis(isocyanatomethyl)-2-methyl-1,3-dithiolane; an aromatic isocyanate compound such as bis(isocyanatoethyl)benzene, bis(isocyanatopropyl)benzene, bis(isocyanatobutyl)benzene, bis(isocyanatomethyl)naphthalene, bis(isocyanatomethyl) diphenyl ether, phenylene diisocyanate, ethylphenylene diisocyanate, isopropylphenylene diisocyanate, dimethylphenylene diisocyanate, diethylphenylene diisocyanate, diisopropylphenylene diisocyanate, trimethylbenzene triisocyanate, benzene triisocyanate, biphenyl diisocyanate, toluene diisocyanate, toluidine diisocyanate, 4,4-diphenylmethane diisocyanate, 3,3-dimethyldiphenylmethane-4,4-diisocyanate, bibenzyl-4,4-diisocyanate, bis(isocyanatophenyl)ethylene, 3,3-dimethoxybiphenyl-4,4-diisocyanate, hexahydrobenzene diisocyanate, hexahydrodiphenylmethane-4,4-diisocyanate, o-xylylene diisocyanate, m-xylylene diisocyanate, *p*-xylylene diisocyanate, xylylene diisocyanate, X-xylylene diisocyanate, 1,3-bis(isocyanatomethyl)cyclohexane, diphenyl sulfide-2,4-diisocyanate, diphenyl sulfide-4,4-diisocyanate, 3,3-dimethoxy-4,4-diisocyanatodibenzyl thioether, bis(4-isocyanatomethylbenzene) sulfide, 4,4-methoxybenzenethioethylene glycol-3,3-diisocyanate, diphenyl disulfide-4,4-diisocyanate, 2,2-dimethyldiphenyl disulfide-5,5-diisocyanate, 3,3-dimethyldiphenyl disulfide-5,5-diisocyanate, 3,3-dimethyldiphenyl disulfide-6,6-diisocyanate, 4,4-dimethyldiphenyl disulfide-5,5-diisocyanate, 3,3-dimethoxydiphenyl disulfide-4,4-diisocyanate, 4,4-dimethoxydiphenyl disulfide-3,3-diisocyanate; and a mixture thereof.

More specifically, 1,3-bis(isocyanatomethyl)cyclohexane, hexamethylene diisocyanate, isophorone diisocyanate, xylylene diisocyanate, toluene diisocyanate, or the like may be used as the isocyanate.

The polymerizable composition may further comprise such additives as an internal mold release agent, an ultraviolet absorber, a polymerization initiator, a heat stabilizer, a color compensator, a chain extender, a crosslinking agent, a light stabilizer, an antioxidant, a filler, and the like.

The internal release agent may include a fluorine-based nonionic surfactant having a perfluoroalkyl group, a hydroxyalkyl group, or a phosphate ester group; a silicone-based nonionic surfactant having a dimethylpolysiloxane group, a hydroxyalkyl group, or a phosphate ester group; an alkyl quaternary ammonium salt such as trimethylcetylammonium salt, trimethylstearylammonium salt, dimethylethylcetylammonium salt, triethyldodecylammonium salt, trioctylmethylammonium salt, and diethylcyclohexadodecylammonium salt; and an acidic phosphate ester. It may be used alone or in combination of two or more.

As the ultraviolet absorber, benzophenone, benzotriazole, salicylate, cyanoacrylate, oxanilide, or the like may be used.

As the polymerization initiator, an amine-based compound, a phosphorus compound, an organotin compound, an organocopper compound, an organogallium compound, an organozirconium compound, an organoiron compound, an organozinc compound, organoaluminum compound, or the like may be used.

As the heat stabilizer, a metal fatty acid salt, a phosphorus compound, a lead compound, or an organotin compound may be used alone or in combination of two or more.

In addition, there is provided an optical material, which comprises a polythiourethane formed by polymerizing the polymerizable composition according to the invention.

The optical material is formed by polymerizing the polythiol composition and an isocyanate.

The polythiourethane as a raw material of the optical material according to the embodiment is prepared by polymerizing (and curing) the polythiol composition and an isocyanate.

The molar ratio of SH group/NCO group in the polymerization reaction may be 0.5 to 3.0, more specifically 0.8 to 1.3.

Further, a reaction catalyst, which is conventionally used in the production of polythiourethane, may be employed in order to control the reaction rate in the polymerization reaction.

As the curing catalyst (or polymerization initiator), a tin-based catalyst may be used. For example, dibutyl tin dichloride, dibutyl tin dilaurate, dimethyl tin dichloride, or the like may be used.

More specifically, the optical material according to the invention is prepared by molding the polythiol composition and an isocyanate while they are polymerized (and cured).

The optical material obtained using the polythiol composition according to the invention has excellent optical properties. Therefore, the optical material can be advantageously used as eyeglass lenses, camera lenses, and the like. The optical material may preferably be a polythiourethane-based lens, i.e., a plastic optical lens.

In addition, there is provided a process for preparing an optical lens using the polythiol composition prepared according to the process as described above.

The process for preparing an optical lens according to the invention comprises (1) preparing a composition comprising a bi- or higher-functional polythiol; (2) treating the composition with a reducing agent; and (3) mixing the composition with an isocyanate and thermally curing the mixture in a mold.

The steps (1) and (2) in the process for preparing an optical lens are carried out according to the conditions and procedures as described above with regard to the steps (1) and (2) of the process for preparing a polythiol composition.

Thereafter, as in the above step (3), the polythiol composition is mixed with an isocyanate and thermally cured in a mold.

For this purpose, the polythiol composition is mixed with an isocyanate to produce a polymerizable composition, which is degassed under reduced pressures and then injected into a mold for molding an optical material. Such degassing and mold injection may be carried out in a temperature range of, for example, 20 to 40°C.

Once the composition is injected into the mold, polymerization is usually carried out by gradually heating the composition from a low temperature to a high temperature. The polymerization temperature may be, for example, 30 to 150°C, more particularly 40 to 130°C. Further, a reaction catalyst, which is conventionally used in the production of polythiourethane, may be employed in order to control the reaction rate. The specific types thereof are as exemplified above.

The molded article obtained as a result is released from the mold to thereby obtain a final optical lens.

The optical lens thus prepared is colorless, transparent, and excellent in such optical characteristics as refractive index, Abbe number, and the like.

The optical lens may have a refractive index in the range of 1.56 to 1.78, more specifically in the range of 1.58 to 1.76, in the range of 1.60 to 1.78, in the range of 1.60 to 1.76, in the range of 1.65 to 1.75, or in the range of 1.69 to 1.75.

The optical lens may have an Abbe number of 20 or more, more specifically 30 or more. For example, the Abbe number of the optical lens may be in the range of 20 to 50, in the range of 25 to 50, in the range of 30 to 45, or in the range of 30 to 43.

The optical lens may have a light transmittance, for example, a light transmittance at a wavelength of 550 nm of 70% to 99.9%, more specifically 75% to 99.0% or 80.0% to 95.0%.

The optical lens may have a yellow index (YI) of 25 or less or 20 or less, specifically in the range of 1 to 25, in the range of 1 to 20, in the range of 3 to 20, or in the range of 5 to 15.

The optical lens may have a glass transition temperature (Tg) of 70°C or more, 80°C or more, or 90°C or more, specifically in the range of 70 to 130°C, in the range of 80 to 120°C, or in the range of 95 to 115°C.

According to a preferred example, the optical lens may have a yellow index (YI) of 1 to 20 and a light transmittance at a wavelength of 550 nm of 85 to 99%.

Further, in such event, the optical lens may have an Abbe number of 30 to 45 and a glass transition temperature (Tg) of 80 to 120°C.

### Example

Hereinafter, the more detailed examples are presented.

### Preparation Example 1: preparation of a polythiol composition

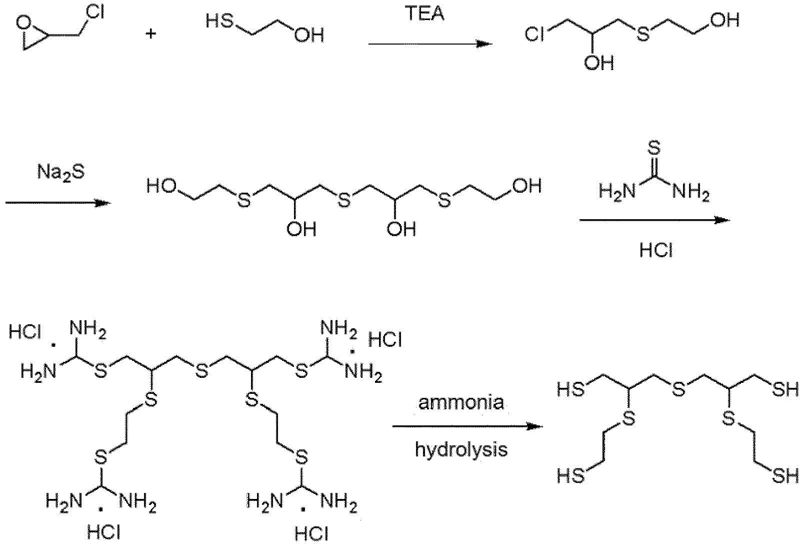

While a mixture of 78.10 g (2 moles) of 2-mercaptoethanol and 2.0 g of triethylamine was maintained at 35 to 45°C, 92.5 g (1 mole) of epichlorohydrin was added thereto dropwise for 1 hours, followed by aging thereof for 1 hour at 40°C. While this reaction solution was maintained at 40°C, an aqueous solution of Na₂S·9H₂O (0.5 mole) dissolved in 100 g of purified water was slowly added dropwise thereto for 1 hour, followed by aging thereof for 1 hour. 303.8 g (3.3 moles) of hydrochloric acid (36%) and 190.3 g (2.5 moles) of thiourea were added to the reaction resultant. While the reaction mixture was stirred, it was heated at 110°C for 9 hours. After the reaction mixture was cooled to room temperature, 400 mL of toluene was added thereto, and 306.5 g (4.5 moles) of aqueous ammonia (25%) was slowly added thereto for the hydrolysis. The organic layer thus obtained was washed twice with 36% hydrochloric acid, 100 mL of water, 100 mL of diluted ammonia, and 100 mL of water to obtain a composition containing a polythiol.

### Comparative Example 1

The organic solvent was removed from the polythiol composition obtained in Preparation Example 1 under the conditions of 60°C and 1333.224 Pa (10 Torr) or less without adding any reducing agent or nitrogen gas to the composition.

### Example 1 (reference example): Removal of active oxygen from a polythiol composition (or treatment with an inert gas)

Active oxygen and the organic solvent were removed from the polythiol composition obtained in Preparation Example 1 while nitrogen (N₂) gas was continuously bubbled at 60°C and 1333.224 Pa (10 Torr) or less without adding any reducing agent to the composition, and the composition was then packaged.

### Example 2: Removal of active oxygen from a polythiol composition (or treatment with a reducing agent)

1 part by weight of sodium borohydride (NaBH4) was added to 100 parts by weight of the polythiol composition obtained in Preparation Example 1, and active oxygen was removed by stirring it at 60°C for 3 hours. Subsequently, the organic solvent was removed while the pressure was lowered to 1333.224 Pa (10 Torr) or less at 60°C. The reducing agent was removed with a 0.2 µm filter, and the composition was then packaged.

### Example 3: Removal of active oxygen from a polythiol composition (or treatment with a reducing agent)

5 parts by weight of lithium aluminum hydride (LiAlH₄) was added to 100 parts by weight of the polythiol composition obtained in Preparation Example 1, and active oxygen was removed by stirring it at 60°C for 3 hours. Subsequently, the organic solvent was removed while the pressure was lowered to 1333.224 Pa (10 Torr) or less at 60°C. The reducing agent was removed with a 0.2 µm filter, and the composition was then packaged.

### Example 4: Removal of active oxygen from a polythiol composition (or treatment with a reducing agent)

5 parts by weight of diisobutyl aluminum hydride (DIBAL-H) was added to 100 parts by weight of the polythiol composition obtained in Preparation Example 1, and active oxygen was removed by stirring it at 60°C for 3 hours. Subsequently, the organic solvent and the reducing agent were removed at the same time while the pressure was lowered to 1333.224 Pa (10 Torr) or less at 60°C. The composition was then packaged.

### Example 5: Removal of active oxygen from a polythiol composition (or treatment with a reducing agent)

2 parts by weight of sodium dithionate (Na₂S₂O₆) was added to 100 parts by weight of the polythiol composition obtained in Preparation Example 1, and active oxygen was removed by stirring it at 60°C for 3 hours. Subsequently, the organic solvent was removed while the pressure was lowered to 1333.224 Pa (10 Torr) or less at 60°C_{.} The reducing agent was removed with a 0.2 µm filter, and the composition was then packaged.

### Example 6 (reference example): Removal of active oxygen from a polythiol composition (or treatment with a reducing agent)

1 part by weight of oxalic acid (C₂H₂O₄) was added to 100 parts by weight of the polythiol composition obtained in Preparation Example 1, and active oxygen was removed by stirring it at 25°C for 1 hour. Subsequently, the organic solvent was removed while the pressure was lowered to 1333.224 Pa (10 Torr) or less at 25°C for 3 hours and then the temperature was raised to 60°C at a pressure of 1333.224 Pa (10 Torr) or less for 3 hours. The reducing agent was removed with a 0.2 µm filter, and the composition was then packaged.

### Example 7: Removal of active oxygen from a polythiol composition (or treatment with a reducing agent)

2 parts by weight of 2,2-diphenyl-1-picrylhydrazide (DPPH) was added to 100 parts by weight of the polythiol composition obtained in Preparation Example 1, and active oxygen was removed by stirring it at 60°C for 3 hours. Subsequently, the organic solvent was removed while the pressure was lowered to 1333.224 Pa (10 Torr) or less at 60°C. The reducing agent was removed with a 0.2 µm filter, and the composition was then packaged.

### Test methods

The polythiol compositions prepared in the Examples and the Comparative Examples were tested in the following manner. The results are summarized in Table 1.

### (1) Measurement of the content of active oxygen (ppm)

A 25 mL flask was purged with nitrogen for 2 minutes, to which about 3 g of the polythiol composition was charged, followed by purging with nitrogen again. 20 mL of ferrous thiocyanate was added to the sample flask and a flask for a blank test, respectively, diluted with methanol, and reacted for 5 minutes. The absorbance of distilled water, the absorbance of the sample, and the absorbance of the blank test solution at a wavelength of 500 nm were measured using a UV-Vis spectrophotometer (Cary50conc, Varian) and a quartz cell (10 mm × 10 mm). The absorbance of the blank test solution was subtracted from the measured absorbance of the sample to calculate the actual absorbance. The amount of active oxygen was obtained using a calibration curve.

### (2) Measurement of the color (b*)

The color of the polythiol composition was measured using a UV-Vis spectrophotometer (Lambda-365, PerkinElmer). Specifically, the color was measured at intervals of 1 nm in a wavelength range of 380 to 780 nm using a quartz cell (10 mm × 10 mm) and a light source of D65/10°.

### (3) Measurement of the viscosity

The viscosity of the polythiol composition was measured using a viscometer (DV3T, Brookfield) under the conditions of CP-51 (cone type) and 10.0 rpm.

### (4) Measurement of the amount of byproducts (% by weight)

The polythiol sample was subjected to gel permeation chromatography (GPC). The amount of byproducts was calculated as a sum of the components that had a shorter elution time (i.e., that have a larger molecular weight) than that of the polythiol as a main component. The content of byproducts was measured immediately after the synthesis of the polythiol and after 6 months of storage.

**[Table 1]**

| | Treatment | Evaluation of polythiol composition | | | | | | |
|---|---|---|---|---|---|---|---|---|
| | | Active oxygen content (ppm) | Color (b*) | | Viscosity (cP) | | Byproducts (wt. %) | |
| | | | Right after synthesis | After 6 months | Right after synthesis | After 6 months | Right after synthesis | After 6 months |
| C. Ex. 1 | None | 230 | 1.9 | 2.7 | 220 | 242 | 27 | 35 |
| Ex. 1 (Referen ce) | N₂ | 80 | 1.2 | 1.4 | 203 | 210 | 18 | 20 |
| Ex. 2 | NaBH₄ | 6 | 0.6 | 0.5 | 190 | 193 | 13 | 13 |
| Ex. 3 | LiAlH₄ | 3 | 0.8 | 0.9 | 192 | 195 | 16 | 16 |
| Ex. 4 | DIBAL-H | 8 | 0.5 | 0.4 | 194 | 190 | 14 | 14 |
| Ex. 5 | Na₂S₂O₃ | 5 | 0.7 | 0.7 | 193 | 195 | 16 | 16 |
| Ex. 6 (Referen ce) | Oxalic acid | 7 | 0.6 | 0.7 | 199 | 197 | 13 | 13 |
| Ex. 7 | DPPH | 9 | 0.5 | 0.7 | 191 | 193 | 16 | 16 |

As shown in Table 1, the polythiol composition of Comparative Example 1, which had a content of active oxygen exceeding 200 ppm since it had not been treated, had a poor color b * value, an increased viscosity, and an increased content of byproducts after storage for 6 months, resulting in a deteriorate in the quality.

In contrast, the polythiol compositions of Examples 1 to 7, in which the content of active oxygen had been reduced to 200 ppm or less, had color b* values, viscosities, and contents of byproducts that were hardly changed even after storage for 6 months.

In particular, in the polythiol compositions of Examples 2 to 7, in which the content of active oxygen had been greatly reduced by treatment with a reducing agent, the color b* value was 1.0 or less, the viscosity was 200 cP or less, and the content of byproducts was 20% by weight or less. Thus, the quality of the compositions was greatly excellent.

## Claims

1. A polythiol composition, which comprises: a bi- or higher-functional polythiol; and a reducing agent, which comprises the reducing agent in an amount of 500 ppm or less, and wherein the reducing agent is at least one selected from the group consisting of lithium aluminum hydride (LiAlH₄), sodium amalgam (Na(Hg)), zinc amalgam (Zn(Hg)), diborane (B₂H₆), sodium borohydride (NaBH₄), iron (II) sulfate (FeSO₄), tin (II) chloride (SnCl₂), sodium dithionate (Na₂S₂O₆), sodium thiosulfate (Na₂S₂O₃), ammonium thiosulfate ((NH₄)₂S₂O₃), diisobutyl aluminum hydride (DIBAL-H), formic acid (HCOOH), ascorbic acid (C₆H₈O₆), reducing sugars, phosphites, hypophosphites, dithiothreitol (DTT), tris-2-carboxyethylphosphine hydrochloride (TCEP), 2,2-diphenyl-1-picrylhydrazide (DPPH), butylated hydroxytoluene (BHT), di-tert-butyl-4-methylphenol, and 2,4-dimethyl-6-tert-butylphenol.

2. A process for preparing the composition of claim 1, which comprises:
preparing a composition comprising a bi- or higher-functional polythiol; and
treating the composition with a reducing agent;
wherein the treatment with the reducing agent is carried out by introducing the reducing agent into the composition and then removing the reducing agent from the composition; and
wherein the reducing agent is at least one selected from the group consisting of lithium aluminum hydride (LiAlH₄), sodium amalgam (Na(Hg)), zinc amalgam (Zn(Hg)), diborane (B₂H₆), sodium borohydride (NaBH₄), iron (II) sulfate (FeSO₄), tin (II) chloride (SnCl₂), sodium dithionate (Na₂S₂O₆), sodium thiosulfate (Na₂S₂O₃), ammonium thiosulfate ((NH₄)₂S₂O₃), diisobutyl aluminum hydride (DIBAL-H), formic acid (HCOOH), ascorbic acid (C₆H₈O₆), reducing sugars, phosphites, hypophosphites, dithiothreitol (DTT), tris-2-carboxyethylphosphine hydrochloride (TCEP), 2,2-diphenyl-1-picrylhydrazide (DPPH), butylated hydroxytoluene (BHT), di-tert-butyl-4-methylphenol, and 2,4-dimethyl-6-tert-butylphenol.

3. A polymerizable composition, which comprises: the polythiol composition according to claim 1; and an isocyanate.

4. An optical material, which comprises: a polythiourethane formed by polymerizing the polythiol composition according to claim 1; and an isocyanate.

5. A process for preparing an optical lens, which comprises:
preparing a polythiol composition according to the process of claim 2; and
mixing the composition with an isocyanate and thermally curing the mixture in a mold.

## Patentansprüche

1. Polythiolzusammensetzung, die ein bi- oder höherfunktionelles Polythiol und ein Reduktionsmittel umfasst, wobei sie das Reduktionsmittel in einer Menge von 500 ppm oder weniger umfasst, und wobei das Reduktionsmittel mindestens eines ist, das ausgewählt ist aus der Gruppe, bestehend aus Lithiumaluminiumhydrid (LiAlH₄), Natriumamalgam (Na(Hg)), Zinkamalgam (Zn(Hg)), Diboran (B₂H₆), Natriumborhydrid (NaBH₄), Eisen-(II)-Sulfat (FeSO₄), Zinn-(II)-Chlorid (SnCl₂), Natriumdithionat (Na₂S₂O₆), Natriumthiosulfat (Na₂S₂O₃), Ammoniumthiosulfat ((NH₄)₂S₂O₃), Diisobutylaluminiumhydrid (DIBAL-H), Ameisensäure (HCOOH), Ascorbinsäure (C₆H₈O₆), reduzierenden Zuckern, Phosphiten, Hypophosphiten, Dithiothreitol (DTT), Tris-2-carboxyethylphosphinhydrochlorid (TCEP), 2,2-Diphenyl-1-picrylhydrazid (DPPH), butyliertem Hydroxytoluol (BHT), Di-tert-butyl-4-methylphenol und 2,4-Dimethyl-6-tert-butylphenol.

2. Verfahren zum Herstellen der Zusammensetzung nach Anspruch 1, das Folgendes umfasst:
Herstellen einer Zusammensetzung, umfassend ein bi- oder höherfunktionelles Polythiol; und
Behandeln der Zusammensetzung mit einem Reduktionsmittel;
wobei die Behandlung mit dem Reduktionsmittel durch Einbringen des Reduktionsmittels in die Zusammensetzung und anschließendes Entfernen des Reduktionsmittels aus der Zusammensetzung durchgeführt wird; und
wobei das Reduktionsmittel mindestes eines ist, das ausgewählt ist aus der Gruppe, bestehend aus Lithiumaluminiumhydrid (LiAlH₄), Natriumamalgam (Na(Hg)), Zinkamalgam (Zn(Hg)), Diboran (B₂H₆), Natriumborhydrid (NaBH₄), Eisen-(II)-Sulfat (FeSO₄), Zinn-(II)-Chlorid (SnCl₂), Natriumdithionat (Na₂S₂O₆), Natriumthiosulfat (Na₂S₂O₃), Ammoniumthiosulfat ((NH₄)₂S₂O₃), Diisobutylaluminiumhydrid (DIBAL-H), Ameisensäure (HCOOH), Ascorbinsäure (C₆H₈O₆), reduzierenden Zucker, Phosphiten, Hypophosphiten, Dithiothreitol (DTT), Tris-2-carboxyethylphosphinhydrochlorid (TCEP), 2,2-Diphenyl-1-picrylhydrazid (DPPH), butyliertem Hydroxytoluol (BHT), Di-tert-butyl-4-methylphenol und 2,4-Dimethyl-6-tert-butylphenol.

3. Polymerisierbare Zusammensetzung, die die Polythiolzusammensetzung nach Anspruch 1 und ein Isocyanat umfasst.

4. Optisches Material, das ein durch Polymerisieren der Polythiolzusammensetzung nach Anspruch 1 gebildetes Polythiourethan und ein Isocyanat umfasst.

5. Verfahren zum Herstellen einer optischen Linse, das Folgendes umfasst:
Herstellen einer Polythiolzusammensetzung gemäß dem Verfahren nach Anspruch 2; und
Mischen der Zusammensetzung mit einem Isocyanat und Wärmehärten des Gemischs in einer Form.

## Revendications

1. Composition de polythiol, qui comprend : un polythiol bifonctionnel ou supérieur ; et un agent de réduction, qui comprend l'agent de réduction en une quantité de 500 ppm ou moins, et dans laquelle l'agent de réduction est au moins un choisi dans le groupe consistant en l'hydrure de lithium et d'aluminium (LiAlH₄), l'amalgame de sodium (Na(Hg)), l'amalgame de zinc (Zn(Hg)), le diborane (B₂H₆), le borohydrure de sodium (NaBH₄), le sulfate de fer (II) (FeSO₄), le chlorure d'étain (II) (SnCl₂), le dithionate de sodium (Na₂S₂O₆), le thiosulfate de sodium (Na₂S₂O₃), le thiosulfate d'ammonium ((NH₄)₂S₂O₃), l'hydrure de diisobutylaluminium (DIBAL-H), l'acide formique (HCOOH), l'acide ascorbique (C₆H₈O₆), les sucres réducteurs, les phosphites, les hypophosphites, le dithiothréitol (DTT), le chlorhydrate de tris-2-carboxyéthylphosphine (TCEP), le 2,2-diphényl-1-picrylhydrazide (DPPH), l'hydroxytoluène butylé (BHT), le di-tert-butyl-4-méthylphénol et le 2,4-diméthyl-6-tert-butyl phénol.

2. Procédé de préparation de la composition de la revendication 1, qui comprend :
la préparation d'une composition comprenant un polythiol bifonctionnel ou supérieur ;
le traitement de la composition avec un agent de réduction ;
dans lequel le traitement avec l'agent de réduction est réalisé par introduction de l'agent de réduction dans la composition et ensuite élimination de l'agent de réduction de la composition ; et
dans lequel l'agent de réduction est au moins un choisi dans le groupe consistant en l'hydrure de lithium et d'aluminium (LiAlH₄), l'amalgame de sodium (Na(Hg)), l'amalgame de zinc (Zn(Hg)), le diborane (B₂H₆), le borohydrure de sodium (NaBH₄), le sulfate de fer (II) (FeSO₄), le chlorure d'étain (II) (SnCl₂), le dithionate de sodium (Na₂S₂O₆), le thiosulfate de sodium (Na₂S₂O₃), le thiosulfate d'ammonium ((NH₄)₂S₂O₃), l'hydrure de diisobutylaluminium (DIBAL-H), l'acide formique (HCOOH), l'acide ascorbique (C₆H₈O₆), les sucres réducteurs, les phosphites, les hypophosphites, le dithiothréitol (DTT), le chlorhydrate de tris-2-carboxyéthylphosphine (TCEP), le 2,2-diphényl-1-picrylhydrazide (DPPH), l'hydroxytoluène butylé (BHT), le di-tert-butyl-4-méthylphénol et le 2,4-diméthyl-6-tert-butylphénol.

3. Composition polymérisable, qui comprend : la composition de polythiol selon la revendication 1; et un isocyanate.

4. Matériau optique, qui comprend : un polythiouréthane formé par polymérisation de la composition de polythiol selon la revendication 1 ; et un isocyanate.

5. Procédé de préparation d'une lentille optique, qui comprend :
la préparation d'une composition de polythiol selon le procédé de la revendication 2 ; et
le mélange de la composition avec un isocyanate et le durcissement thermique du mélange dans un moule.
